# EUROPEAN PATENT APPLICATION

(11) **EP 4 657 457 A1**
(43) Date of publication of application: **03.12.2025**
(21) Application number: 25169029.3
(22) Date of filing: 08.04.2025
(51) Int. Cl.: G16H 40/67, A61B 5/145

(54) **METHOD OF OUTPUTTNG BLOOD GLUCOSE DATA**

(30) Priority: 28.05.2024 KR 20240069473
(71) Applicant: i-SENS, INC., Seoul 06646 (KR)
(72) Inventor: KIM, Kyu Jin, Incheon (KR)
(74) Representative: HGF

(57) **Abstract**

One embodiment may provide a method of outputting blood glucose data including obtaining biometric information in response to an advertisement transmitted by a sensor transmitter, generating the biometric information as output, in the case that a communication failure occurs, indicating a data gap during a period of the communication failure, and if the communication failure is resolved, filling the data gap during a period from a time point when the communication failure is resolved to one advertisement which arrives after the communication failure is resolved.

## Description

### CROSS-REFERENCE TO RELATED PATENT APPLICATION

The present application claims priority under 35 U.S.C. § 119(a) to Korean patent application number 10-2024-0069473 filed on May 28, 2024, in the Korean Intellectual Property Office, the entire disclosure of which is incorporated by reference herein.

### BACKGROUND OF THE INVENTION

### 1. Field

Embodiments of the present disclosure relate to outputting blood glucose data from a glucose monitoring system, and more specifically, to a technology for, in the case that a communication failure occurs, receiving and outputting biometric information stored in a sensor transmitter again after the communication failure is resolved.

### 2. Description of the Related Art

Recently, with the advancement of medical technology, various medical devices that are attached to a user's body have been developed and sold. A medical device attached to the user's body may be useful for monitoring biometric information or providing treatment by being attached to the skin of a chronic disease patient.

For example, chronic diseases such as diabetes require continuous management, and a medical device that is attached to the skin and measures glucose may be used to monitor glucose in diabetic patients. Diabetes is characterized by almost no noticeable symptoms in the early stages, but as the disease progresses, symptoms specific to diabetes appear, such as polydipsia, polyphagia, polyuria, weight loss, general malaise, itchy skin, and wounds on the hands and feet that do not heal and persist for a long time. As diabetes progresses further, complications such as vision impairment, high blood pressure, kidney disease, stroke, periodontal disease, muscle spasms, neuralgia, and gangrene appear. In order to diagnose diabetes and manage it to prevent it from developing into complications, systematic glucose measurement and treatment must be carried out together.

For diabetic patients and people who have not developed diabetes but have more sugar than normal detected in their blood, many medical device manufacturers provide various types of glucose meters that may measure glucose.

There are two types of glucose meters: one that measures blood glucose levels on a one-time basis by drawing blood from the user's fingertip, and one that measures blood glucose levels continuously by attaching the meter to the user's stomach or arm.

In the case of diabetic patients, they generally go back and forth between hyperglycemia and hypoglycemia, and emergency situations occur during hypoglycemia, and loss of consciousness or prolonged hypoglycemia without sugar supply may result in death. Therefore, immediate detection of hypoglycemia is very important for diabetic patients, but glucose meters that measure glucose intermittently have limitations in accurately detecting this condition.

Recently, to overcome these limitations, a continuous glucose monitoring system (CGMS), which is inserted into the human body and measures glucose levels at intervals of several minutes, has been developed and used. In order to minimize the user's pain and resistance following blood collection, the CGMS may measure glucose continuously after inserting a needle-shaped transdermal sensor into areas where pain is relatively less, such as the stomach or arm.

The CGMS includes a sensor transmitter that is inserted into the user's skin to measure glucose in the body and transmit the measured glucose level, and a terminal that outputs the received glucose level.

In the CGMS, the sensor transmitter and the communication terminal transmit and receive blood glucose information wired or wirelessly, and the terminal must continuously receive data packets containing blood glucose information from the sensor transmitter. However, if the terminal fails to continuously receive glucose information from the sensor transmitter due to a temporary communication interruption between the sensor transmitter and the terminal or the user's inexperienced actions, or if the sensor transmitter and the terminal are separated from each other by a distance that prevents them from communicating with each other for a considerable period of time, the terminal may fail to receive the user's glucose information during that time.

As such, if the terminal cannot receive glucose information from the sensor transmitter, the terminal needs to notify the user that there is unreceived glucose information and receive the unreceived glucose information.

### SUMMARY OF THE INVENTION

Against this background, an object of embodiments of the present disclosure is to provide a method of indicating a data gap due to a communication failure and receiving and outputting biometric information stored in a sensor transmitter again after the communication failure is resolved.

In order to achieve the above described object, an embodiment may provide a method of outputting blood glucose data including: obtaining biometric information in response to an advertisement transmitted by a sensor transmitter; generating the biometric information as output; in the case that a communication failure occurs, indicating a data gap during a period of the communication failure; and if the communication failure is resolved, filling the data gap during a period from a time point when the communication failure is resolved to one advertisement which arrives after the communication failure is resolved.

In the method, the filling of the data gap may include outputting biometric information stored in the sensor transmitter during the communication failure to fill the data gap after the communication failure is resolved.

In the method, the filling of the data gap may include outputting biometric information stored in the sensor transmitter during the communication failure to fill the data gap at an advertisement which arrives first after the communication failure is resolved.

The method may further include storing biometric information stored in the sensor transmitter in a terminal, and the filling of the data gap may include sequentially outputting biometric information stored in the terminal after last output biometric information.

In the method, the indicating of the data gap may include, in the case that the communication failure occurs, outputting the data gap while the biometric information stored in the terminal exists.

In the method, the filling of the data gap may include, if the communication failure is resolved, outputting the biometric information stored in the terminal again following the last output biometric information.

In the method, the filling of the data gap may include, after sequentially outputting the biometric information stored in the terminal after the last output biometric information, waiting until an advertisement.

As described above, according to the embodiments, after a communication failure is resolved, by receiving and outputting biometric information stored in a sensor transmitter again at one time point during a predetermined period of time, the biometric information maybe received and provided to the user without loss.

In addition, according to the embodiments, even if there is stored biometric information, the occurrence of the communication failure may be provided to the user in a timely manner by outputting a data gap immediately upon occurrence of the communication failure.

### BRIEF DESCRIPTION OF THE DRAWINGS

FIG. 1 is a diagram for schematically explaining a glucose monitoring system according to an embodiment.
FIG. 2 is a diagram for explaining an applicator for attaching a sensor transmitter to a human body according to an embodiment.
FIG. 3 is a diagram for explaining a process of attaching a sensor transmitter to a human body using an applicator according to an embodiment.
FIG. 4 is a configuration diagram of a sensor transmitter according to an embodiment.
FIG. 5 is a configuration diagram of a terminal according to an embodiment.
FIG. 6 is a diagram illustrating a first example in which biometric information is generated in a sensor transmitter according to an embodiment.
FIG. 7 is a diagram illustrating a second example in which biometric information is generated in a sensor transmitter according to an embodiment.
FIG. 8 is a flowchart for explaining a method of transmitting and receiving biometric information between a sensor transmitter and a terminal according to an embodiment.
FIG. 9 is an example diagram of a user interface provided by a terminal to output a data gap according to an embodiment.
FIG. 10 is a diagram for explaining an example of a terminal outputting biometric information stored in a sensor transmitter after a communication failure is resolved according to an embodiment.
FIG. 11 is a diagram for explaining another example of a terminal outputting biometric information stored in a sensor transmitter according to an embodiment.
FIG. 12 is a flowchart illustrating an example of a method of receiving and outputting biometric information stored in a sensor transmitter by a terminal according to an embodiment.
FIG. 13 is a flowchart illustrating another example of a method of receiving and outputting biometric information stored in a sensor transmitter by a terminal according to an embodiment.

### DETAILED DESCRIPTION

In describing the present disclosure, if it is determined that related known functions may unnecessarily obscure the gist of the present disclosure as they are obvious to those skilled in the art, detailed descriptions thereof will be omitted.

The terms used in the present application are used merely to describe particular embodiments and are not intended to limit the present disclosure. Singular expressions include plural expressions unless the context clearly indicates otherwise. In the present application, it should be understood that terms such as "comprise", "include", or "have" are intended to designate the presence of features, numbers, steps, operations, components, parts, or combinations thereof described in the specification, and they do not preclude the possibility of the presence or addition of one or more other features, numbers, steps, operations, components, parts, or combinations thereof.

Terms such as first and second are merely identifiers used to distinguish identical or corresponding components, and the identical or corresponding components are not limited by terms such as first and second.

Hereinafter, embodiments according to the present disclosure will be described in detail with reference to the accompanying drawings, and in describing with reference to the accompanying drawings, identical or corresponding components will be assigned the same drawing numbers and overlapping descriptions thereof will be omitted.

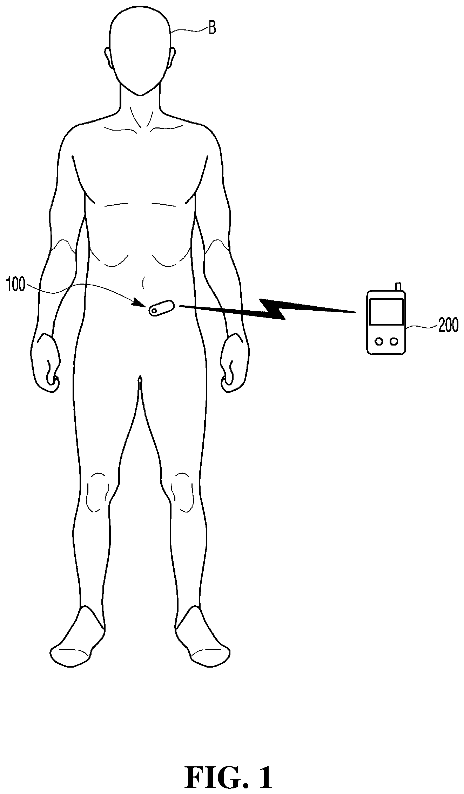

FIG. 1 is a diagram for schematically explaining a glucose monitoring system according to an embodiment.

Referring to FIG. 1, a glucose monitoring system 10 (hereinafter referred to as a "system") according to an embodiment may include a sensor transmitter 100 and a terminal 200.

The sensor transmitter 100 is attached to a human body B, and when the sensor transmitter 100 is attached to the human body B, one end of a sensor of the sensor transmitter 100 is inserted into the skin to periodically extract body fluids from the human body and measure glucose.

The terminal 200 may receive a biosignal including glucose information from the sensor transmitter 100, generate glucose information from the biosignal, and output to the user. The terminal 200 may include, but is not limited to, various devices such as smartphones, mobile phones, tablet PCs, desktops, and laptops, and may have a communication interface capable of communicating with the sensor transmitter 100 and may include a device on which a program or application may be installed.

The sensor transmitter 100 may periodically transmit measured biosignals to the terminal 200 at the request of the terminal 200 or at a set time. For data communication between the sensor transmitter 100 and the terminal 200, the sensor transmitter 100 and the terminal 200 may be connected to each other via a wired connection such as a USB cable or wirelessly using methods such as infrared communication, NFC communication, or Bluetooth.

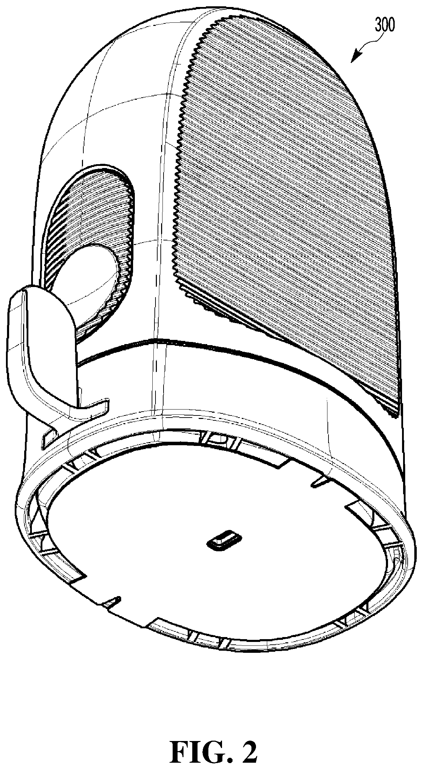

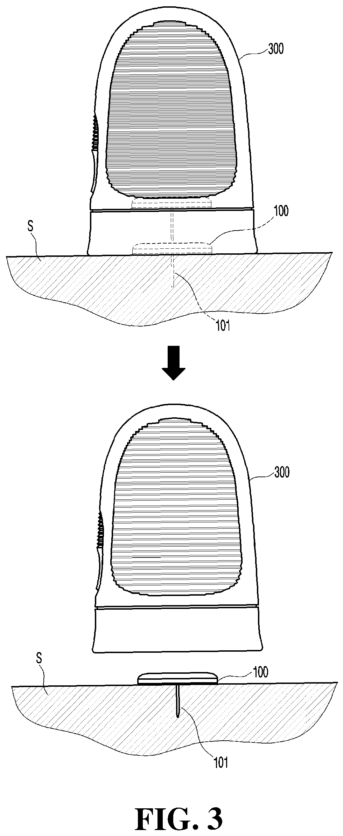

FIG. 2 is a diagram for explaining an applicator for attaching a sensor transmitter to a human body according to an embodiment, and FIG. 3 is a diagram for explaining a process of attaching a sensor transmitter to a human body using an applicator according to an embodiment.

Referring to FIGS. 2 and 3, an applicator 300 according to an embodiment has the sensor transmitter 100 inside and operates to discharge the sensor transmitter 100 to the outside and attach it to a specific body part of the user through manipulation by the user. The applicator 300 is formed in a shape with one side open, and the sensor transmitter 100 is installed in the applicator 300 through the open side of the applicator 300.

When attaching the sensor transmitter 100 to a part of the body using the applicator 300, in order to insert one end of a sensor provided in the sensor transmitter 100 into the skin, the applicator 300 may include a needle (not shown) formed to surround one end of the sensor inside, a first elastic member (not shown) that pushes the needle and one end of the sensor together into the skin, and a second elastic member (not shown) for pulling out only the needle. Through this configuration of the applicator 300, the needle and one end of the sensor may be simultaneously inserted into the skin by decompressing the first elastic member (not shown) disposed in a compressed state inside the applicator 300. When one end of the sensor is inserted into the skin, only the needle is pulled out by decompressing the compressed second elastic member (not shown). The user may safely and easily attach the sensor transmitter 100 to the skin through the applicator 300.

Looking in detail at the process of attaching the applicator 300 to the human body B, with a protective cap (not shown) removed, the open side of the applicator 300 is brought into close contact with the skin S of a specific area of the human body B. When the applicator 300 is operated in this way while the applicator 300 is in close contact with the skin S of the human body B, the sensor transmitter 100 is discharged from the applicator 300 and may be attached to the skin S. Here, one end of the sensor 101 is disposed at the lower part of the sensor transmitter 100, exposed from the sensor transmitter 100, and one end of the sensor 101 may be partially inserted into the skin S through the needle provided in the applicator 300. Therefore, the sensor transmitter 100 may be attached to the skin S with one end of the sensor 101 inserted into the skin S.

Here, an adhesive tape may be provided on the surface of the sensor transmitter 100 in contact with the human body B so that the sensor transmitter 100 may be fixedly attached to the skin S of the human body B. Therefore, when the applicator 300 is separated from the skin S of the human body B, the sensor transmitter 100 may be fixedly attached to the skin S of the human body B by the adhesive tape.

Afterwards, when power is applied to the sensor transmitter 100, the sensor transmitter 100 communicates with the terminal, and the sensor transmitter 100 may transmit biosignals including glucose information to the terminal. The sensor transmitter 100 may generate not only glucose information but also various biometric information, and hereinafter, it will be explained that glucose information is measured as an example of biometric information.

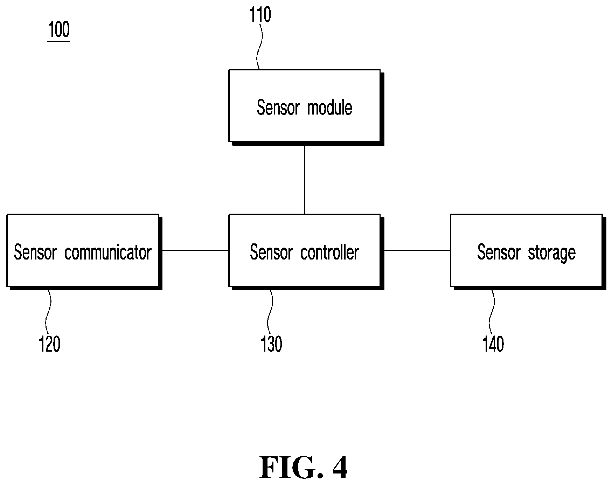

FIG. 4 is a configuration diagram of a sensor transmitter according to an embodiment.

Referring to FIG. 4, the sensor transmitter 100 according to an embodiment may include a sensor module 110, a sensor communicator 120, a sensor controller 130, and a sensor storage 140.

The sensor module 110 may include at least one sensor that is inserted into the human body and senses biomass. At least one sensor may measure biomass and generate biosignals. The biosignal is an analog signal and may include a current value.

The sensor communicator 120 may exchange data or information with the terminal. For example, the sensor communicator 120 may transmit biosignals received from the sensor module 110 or data stored in the sensor storage 140 (for example, biometric information) to the terminal.

The sensor controller 130 may control the overall configuration of the sensor transmitter 100, including the sensor module 110, the sensor storage 140, and the sensor communicator 120. For example, the sensor controller 130 may receive a control signal from the terminal and control the configuration of the sensor transmitter 100 accordingly. In addition, the sensor controller 130 may process biosignals. For example, the sensor controller 130 may convert biosignals into analog or digital form or perform processing to remove noise as needed.

Data or information may be stored in the sensor storage 140. For example, the sensor storage 140 may store data on biomass measured by the sensor module 110, for example, the current value of the biosignal or its digital form data, or data received from the terminal, for example, the command value of the control signal.

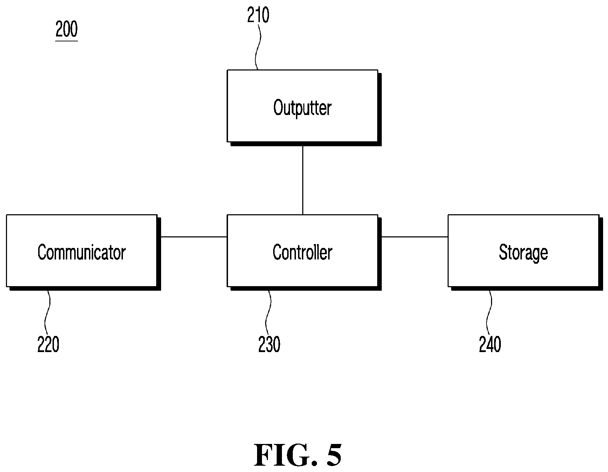

FIG. 5 is a configuration diagram of a terminal according to an embodiment.

Referring to FIG. 5, the terminal 200 according to an embodiment may include an outputter 210, a communicator 220, a controller 230, and a storage 240.

The outputter 210 may output biometric information included in the biosignal, for example, glucose information, so that the user may check it. For example, the outputter 210 may display glucose information as a numerical level (value) or a graph processed from numerical value.

The communicator 220 may communicate with the sensor communicator of the sensor transmitter and exchange data or information. For example, the communicator 220 may receive a biosignal containing information about biomass (i.e., biometric information) measured by the sensor transmitter. Here, the communicator 220 may receive primarily processed biosignals from the sensor transmitter. Preferably, the processed biosignal may include discrete data (discontinuous data) in which a current value, which is an analog signal, is converted into digital data. If the current value is sampled every cycle, digital discrete data may be generated. Alternatively, the communicator 220 may transmit a control signal for controlling the sensor transmitter to the sensor transmitter.

Data or information may be stored in the storage 240. For example, data received from the sensor transmitter, for example, biometric information, may be stored in the storage 240. Here, biometric information may include glucose information, and may include digital data representing current values. Alternatively, data input from the user or environment setting data for setting the operating environment of the terminal may be stored in the storage 240.

The controller 230 may include at least one processor that executes a program for outputting unreceived data in a glucose monitoring system and at least one memory in which the program is stored. The memory and processor included in the controller 230 may be integrated into one chip or may be physically separated.

Memory may be implemented as non-volatile memory devices such as read only memory (ROM), programmable ROM (PROM), erasable programmable ROM (EPROM), electrically erasable programmable ROM (EEPROM), and flash memory, or volatile memory devices such as random access memory (RAM) to store various programs, data, and/or information.

In addition, the controller 230 may generate a glucose value, which is quantified blood glucose information, from biometric information. To this end, the controller 230 may obtain biometric information in the form of a current value from the sensor transmitter, and preprocess and/or process the current value of the biometric information. The controller 230 may first calculate the sensitivity and generate the glucose value according to this sensitivity.

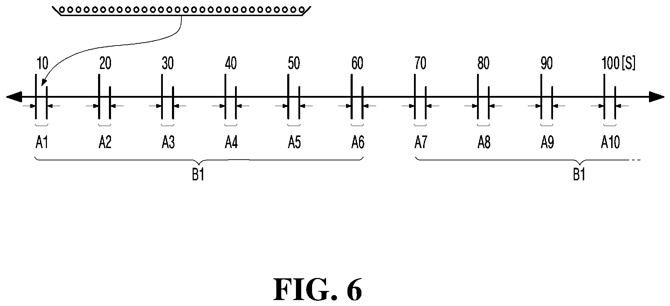

FIG. 6 is a diagram illustrating a first example in which biometric information is generated in a sensor transmitter according to an embodiment.

Referring to FIG. 6, biometric information may be generated in a sensor transmitter according to an embodiment. Specifically, the sensor transmitter may obtain an analog (continuous) biosignal representing a current value at predetermined intervals, and sample the biosignal to generate digital (discontinuous) data representing the current value. The generated data may be processed in the sensor transmitter and biometric information may be generated. Hereinafter, it is described that biometric information is generated by generating and processing digital data from a biosignal in the sensor transmitter, but is not limited thereto, and part or all of the processing may be performed in the sensor transmitter depending on the embodiment.

For example, the sensor transmitter may obtain a biosignal in an analog form, for example, a current value, measure the biosignal every 10 seconds, and process the measured biosignal to generate a single first data. Specifically, the sensor transmitter may measure (sample) a biosignal 30 times every 10 seconds and generate digital data. The sensor transmitter may remove upper data and lower data from the 30 data, calculate an average value (A1) of the remaining data, and determine this average value (A1) as the single first data. The first data, which is the data of the average value (A1) calculated in this way, is generated in 10 second-units, and as illustrated, six average values (A1 to A6), i.e., six first data, may be generated in 1 minute.

In addition, the sensor transmitter may process 30 first data every 300 seconds (5 minutes). The sensor transmitter may generate an average value (B1) again using the six first data (average values (A1 to A6)). In generating the average value (B1), the terminal may remove the upper data and lower data among the six average values (A1 to A6) and generate the average value (B1) of the remaining data. The second data, which is the data of the average value (B1) calculated in this way, is generated in 1 minute-units, and as illustrated, 1 average value (B1), i.e., 1 second data, may be generated in 1 minute.

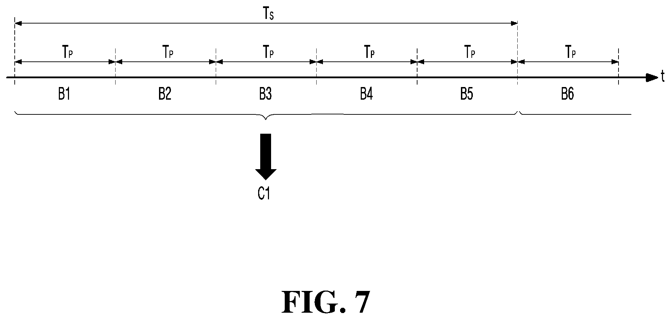

FIG. 7 is a diagram illustrating a second example in which biometric information is generated in a sensor transmitter according to an embodiment.

Referring to FIG. 7, second data may be processed to generate biometric information in a sensor transmitter according to an embodiment. In the above-described example, the sensor transmitter may obtain 5 second data (B1) in 1 minute-units from 6 first data in 10 second-units. In addition, the sensor transmitter may generate an average value (C1) using the 5 second data (average values (B1 to B5)). In generating the average value (C1), the terminal may remove upper data and lower data among the 5 average values (B1 to B5) and generate an average value (C1) of the remaining data. The third data, which is the data of the average value (C1) calculated in this way, is generated in 5-minute units, and as illustrated, 1 average value (C1), i.e., 1 third data, may be generated in 5 minutes.

Here, the terminal may sequentially generate second data (B1 to B5) during a biometric information generation period (Tp) and generate one third data (C1) during a glucose value biometric information generation period (Ts). In the glucose value biometric information generation period (Ts), a glucose value is calculated from the third data (C1) through sensitivity, and in the biometric information generation period (Tp), second data (B1 to B5) that serve as the basis for the third data (C1) may be generated. In the example described above, the biometric information generation period (Tp) may correspond to 1 minute, and the glucose value biometric information generation period (Ts) may correspond to 5 minutes.

In this way, the third data generated in 5-minute units may undergo a noise removal process by a filter. The filtered third data may be converted into biometric information including a glucose value by applying sensitivity, and such biometric information may be output to the user.

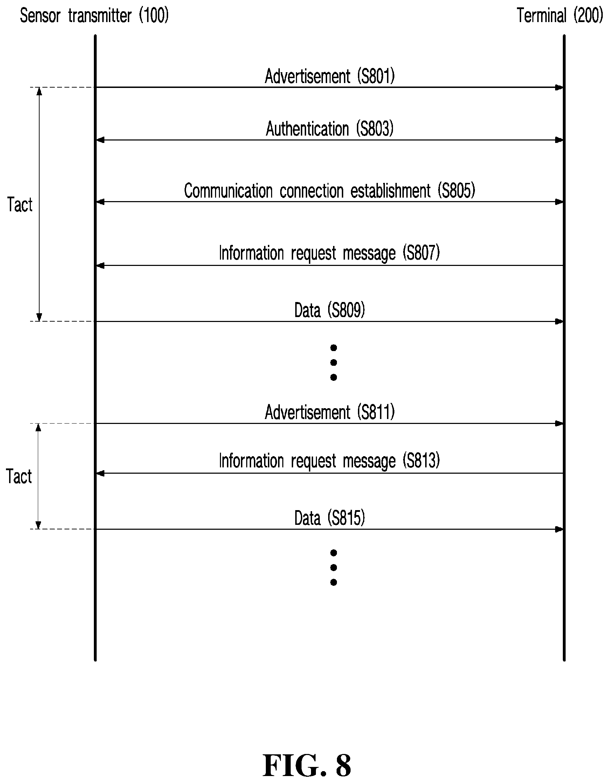

FIG. 8 is a flowchart for explaining a method of transmitting and receiving biometric information between a sensor transmitter and a terminal according to an embodiment.

Referring to FIG. 8, the sensor transmitter 100 and the terminal 200 according to an embodiment may connect communication in order to transmit and receive data including biometric information. Data may be transmitted and received after the communication connection. The sensor transmitter 100 and the terminal 200 may be connected to each other through wired or wireless communication, and may connect communication in a manner such as USB communication, infrared communication, Bluetooth communication, etc.

Specifically, the sensor transmitter 100 and the terminal 200 may transmit and receive differently depending on whether communication is disconnected and a new communication is connected (when connecting initial communication) or only data is transmitted and received after initial communication is connected. First, in a state of communication disconnection, when the sensor transmitter 100 and the terminal 200 establish a new communication connection, the sensor transmitter 100 may advertise to the terminal (step S801). The sensor transmitter 100 may generate a certain signal for advertisement and transmit this advertisement signal to the terminal. Alternatively, the sensor transmitter 100 may perform advertisement by periodically transmitting an advertisement message to the terminal 200. The process of sending this advertisement signal or advertisement message may be called advertisement. The terminal 200 may receive the advertisement signal or the advertisement message and perform authentication with the sensor transmitter 100 (step S803). For example, the sensor transmitter 100 and the terminal 200 may authenticate that they are valid devices through a hash value. Then, the sensor transmitter 100 and the terminal 200 may establish a communication connection (step S805). The communication connection is a state in which data may be transmitted and received immediately without going through a separate initial communication process such as authentication, and the sensor transmitter 100 and the terminal 200 may transmit and receive data in response to an advertisement at any time while the communication connection is being established. The terminal 200 may transmit an information request message to the sensor transmitter 100 to obtain data including biometric information (for example, 30 pieces of first data) (step S807). The sensor transmitter 100 that has received the information request signal or information request message may transmit data including biometric information (for example, 30 pieces of first data) to the terminal 200 in response thereto (step S809).

Once the initial communication is connected, data may be transmitted and received in the communication connection establishment state without a separate authentication process. The sensor transmitter 100 may advertise to the terminal 200 repeatedly during the following operation section (step S811). The repeated advertisement may be performed by transmitting an advertisement signal or an advertisement message to the terminal 200 periodically or aperiodically.

The terminal 200 may send an information request message requesting data transmission in response to the advertisement to the sensor transmitter 100 (step S813). The sensor transmitter 100 may transmit data in response to the information request signal or information request message (step S815).

Here, the terminal 200 receives data from the sensor transmitter 100 from time to time, but the terminal 200 may receive data in response to the advertisement only when the sensor transmitter 100 advertises. The sensor transmitter 100 may send this advertisement signal or advertisement message to the terminal 200 periodically or aperiodically, and the terminal 200 may request and receive data accordingly. **In** addition, the sensor transmitter 100 may not continuously send the advertisement signal or advertisement message, but may transmit only in active mode, i.e., while awake. Accordingly, data transmission and reception between the sensor transmitter 100 and the terminal 200 may be performed only during this active mode period (Tact). The sensor transmitter 100 may stand by without sending any data during the inactive mode, i.e, the non-active mode period.

The terminal 200 may determine whether there is unreceived data (i.e., unreceived biometric information) through an identifier assigned to the biometric information. Since an identifier is assigned to data (or data packets) including biometric information, the terminal 200 may determine whether data has been received from the sensor transmitter 100 through this identifier. For example, biometric information received from the sensor transmitter 100 may be stored in the storage of the terminal 200 and output sequentially. The biometric information is assigned an identifier by the sensor transmitter 100, and this identifier may be a serial number assigned according to the order in which the biometric information is generated by the sensor transmitter 100. The terminal 200 may determine the total number of biometric information received by the terminal 200 through the last identifier of the stored biometric information. The sensor transmitter 100 may also calculate the total number of biometric information to be transmitted to the terminal 200 through the last identifier of the stored biometric information. The sensor transmitter 100 may continuously generate biometric information and assign an identifier to the biometric information regardless of the communication connection with the terminal 200. When the sensor transmitter 100 starts communication with the terminal 200, the sensor transmitter 100 may sequentially send to the terminal 200 biometric information after the last identifier of the biometric information transmitted to the terminal 200. The terminal 200 receives all of the biometric information from the sensor transmitter or when the active mode period (Tact) during which communication is possible ends, the terminal 200 may end communication with the sensor transmitter 100.

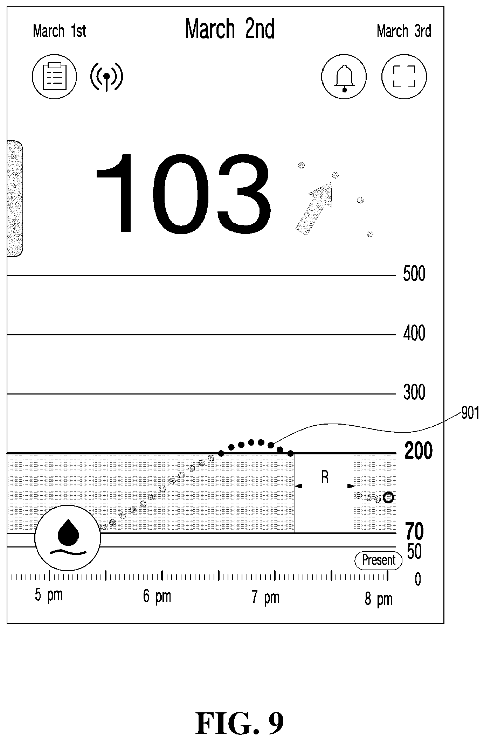

FIG. 9 is an example diagram of a user interface provided by a terminal to output a data gap according to an embodiment.

Referring to FIG. 9, an example of a user interface implemented to output a data gap by a terminal according to an embodiment may be illustrated. The controller of the terminal may execute an application, and the outputter may visually display a user interface implemented by the application. The outputter may obtain biometric information from the controller and display it on the user interface.

The user interface may include an icon including biometric information and a convenient function for providing the same. For example, the icon may indicate a communication state, for example, a Bluetooth communication connection. For example, the icon may indicate a smooth communication state (where a communication connection is established and biometric information is received) as blue, indicate a state in which the terminal does not receive biometric information for a predetermined period of time (a case in which a communication connection is established but biometric information is not received) as red, and indicate a state in which the communication module is turned off or is not connected to the sensor for communication (a case in which no communication connection is established) as gray. Therefore, when the communication module is turned off, the icon may tum gray.

In addition, the user interface may include a trend 901 in order to indicate biometric information. The terminal receives and outputs biometric information from the sensor transmitter at regular intervals, and this biometric information may be output at each interval to form a kind of pattern, i.e., a trend 901. The trend may have a form similar to a graph. The trend 901 may be expressed over an area consisting of the highest and lowest glucose values. If data (biometric information) is not received by the terminal due to a communication failure, data (biometric information) may not be output during that period. A blank area (data gap R) where the data (biometric information) that is not output appears may exist in the trend 901. The data gap R may represent the absence of biometric information due to which the terminal cannot receive and/or output biometric information. If the communication failure is resolved, data may be received from the sensor transmitter to the terminal, biometric information may be output again, and the data gap R may be filled. Even if the sensor transmitter cannot transmit biometric information to the terminal, 12 hours worth of biometric information may be stored. Therefore, if the data gap R is formed within 12 hours, the terminal may receive biometric information that was not received during the communication failure and fill the data gap R.

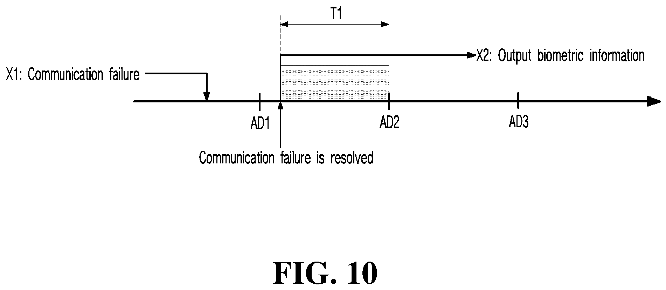

FIG. 10 is a diagram for explaining an example of a terminal outputting biometric information stored in a sensor transmitter after a communication failure is resolved according to an embodiment.

Referring to FIG. 10, an example of a terminal outputting biometric information stored in a sensor transmitter after a communication failure is resolved according to an embodiment may be illustrated. In this example, in the case that a communication failure occurs, the terminal outputs a data gap representing a blank in which biometric information is not output, and if the communication failure is resolved, the terminal may receive and output the biometric information stored in the sensor transmitter again.

Specifically, a communication failure may occur at time point X1 while the sensor transmitter and the terminal are communicating. Here, the communication failure may refer to a state in which data cannot be received due to a communication module (e.g., a Bluetooth communication module) being turned off or due to any failure (e.g., when the distance between the sensor transmitter and the terminal increases) even when the communication module is turned on. The former example corresponds to a case in which the communication connection between the sensor transmitter and the terminal is cut off, and the latter example corresponds to a case in which the terminal stops receiving data from the sensor transmitter while the communication connection is established. The terminal may determine whether there is a communication failure through the operating status of the communication module and whether data is received. If the terminal does not receive data for a predetermined period of time, the terminal may output a data gap.

In addition, the terminal may receive biometric information from the sensor transmitter in response to an advertisement sent by the sensor transmitter. If a communication failure occurs, even if the sensor transmitter transmits a signal or message to the terminal for advertisement, the terminal may not receive the signal or message. Alternatively, even if the signal or message reaches the terminal, the terminal may not be able to transmit the information request message or receive data including biometric information in response to the information request message. At the time point when the sensor transmitter advertises, i.e., at the advertisement timing (AD1, AD2, AD3), each terminal may not be able to receive data from the sensor transmitter as described above.

If no communication failure occurs, the advertisement timing (AD1, AD2, AD3) may, in principle, include the following characteristics. The advertisement timings (AD1, AD2, AD3) may be periodic or aperiodic. When the advertisement timings (AD1, AD2, AD3) are periodic, the advertisement timings (AD1, AD2, AD3) may be formed at 1-minute intervals. At the advertisement timings (AD1, AD2, AD3), i.e., at 1-minute intervals, the sensor transmitter may advertise to the terminal and send a signal or message for this advertisement to the terminal. In addition to the advertisement, the sensor transmitter may transmit data including biometric information to the terminal at the advertisement timings (AD1, AD2, AD3). Strictly speaking, when the sensor transmitter collects data from the sensor for a predetermined period of time, the collected datamay be transmitted to the terminal at any one of the advertisement timings (AD1, AD12, AD3). As in the example described above, the sensor transmitter may collect and process 300 seconds (5 minutes) of data and transmit to the terminal. The sensor transmitter may continuously collect first data in units of 10 seconds, generate second data in units of 60 seconds (1 minute) from the first data, and generate third data in units of 300 seconds (5 minutes) from the second data. When 300 seconds (5 minutes) of data (i.e., the third data) are completed at any one of the advertisement timings (AD1, AD2, AD3), the sensor transmitter may send the 300 seconds (5 minutes) of data to the terminal at once. For example, if the sensor transmitter has already completed 300 seconds (5 minutes) of data at the advertisement timing (AD1), it may only advertise and not transmit data at the advertisement timing (AD2, AD3). If the sensor transmitter should have completed 300 seconds (5 minutes) of data at the advertisement timing (AD1) but did not, it may complete 300 seconds (5 minutes) of data by the next advertisement timing (AD2 or AD3) and transmit it to the terminal. The advertisement timings (AD1, AD2, AD3) are repeated, and other advertisement timings may be formed at 1-minute intervals thereafter. Here, the advertisement timings (AD1, AD2, AD3) may be named the first to third advertisement timings, respectively.

Thereafter, if the communication failure is resolved, the terminal may receive and output the biometric information stored in the sensor transmitter to fill the data gap at one time point during the period (shaded) from the time point when the communication failure is resolved to one advertisement timing which arrives after the communication failure is resolved. During the first period (T1), the terminal may receive and output the biometric information stored in the sensor transmitter at any time point. Preferably, in this example, the terminal may receive and output the biometric information stored in the sensor transmitter immediately after the communication failure is resolved, at time point X2.

Here, if 300 seconds (5 minutes) of data are prepared in the sensor transmitter, the terminal may receive data (biometric information) at the advertisement timing (AD1, AD2, AD3), and thus may wait to receive the biometric information stored in the sensor transmitter until the advertisement timing which arrives first after the communication failure is resolved, i.e., the second advertisement timing (AD2). If there is stored biometric information remaining in the terminal that has not been output yet, it may be outputted first and then wait. In this example, the terminal is described as receiving and outputting the biometric information stored in the sensor transmitter at time point X2, which is the time point when the communication failure is resolved, even if the biometric information stored in the sensor transmitter is output at a time point other than the second advertisement timing (AD2) during the first period (T1), the terminal may similarly wait to receive the biometric information stored in the sensor transmitter until the second advertisement timing (AD2).

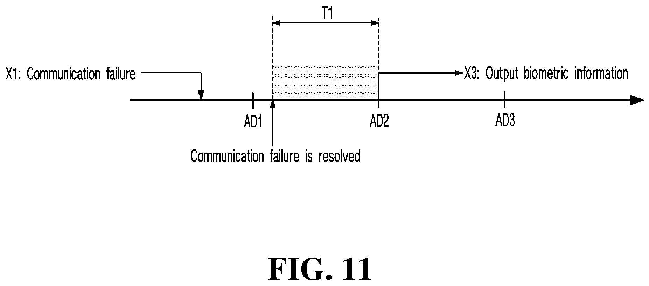

FIG. 11 is a diagram for explaining another example of a terminal outputting biometric information stored in a sensor transmitter according to an embodiment.

Referring to FIG. 11, another example of a terminal outputting biometric information stored in a sensor transmitter according to an embodiment may be illustrated. Unlike an embodiment, in this example, if the communication failure is resolved, the terminal does not output the biometric information stored in the sensor transmitter immediately, i.e., from time point X2, but may receive and output the biometric information stored in the sensor transmitter from the advertisement timing which arrives first, i.e., the second advertisement timing (AD2) or time point X3.

Specifically, a communication failure may occur at time point X1 while the sensor transmitter and the terminal are communicating. If the terminal does not receive data for a predetermined period of time, the terminal may output a data gap indicating the existence of biometric information stored in the sensor transmitter. Thereafter, if the communication failure is resolved, the terminal may output the biometric information stored in the sensor transmitter to fill the data gap at one time point during the period (shaded) from the time point when the communication failure is resolved to one advertisement timing which arrives after the communication failure is resolved. During the first period (T1), the terminal may receive and output the biometric information stored in the sensor transmitter at any time point. Preferably, in this example, the terminal may again receive and output the biometric information stored in the sensor transmitter at one advertisement timing which arrives first after the communication failure is resolved, i.e., the second advertisement timing (AD2).

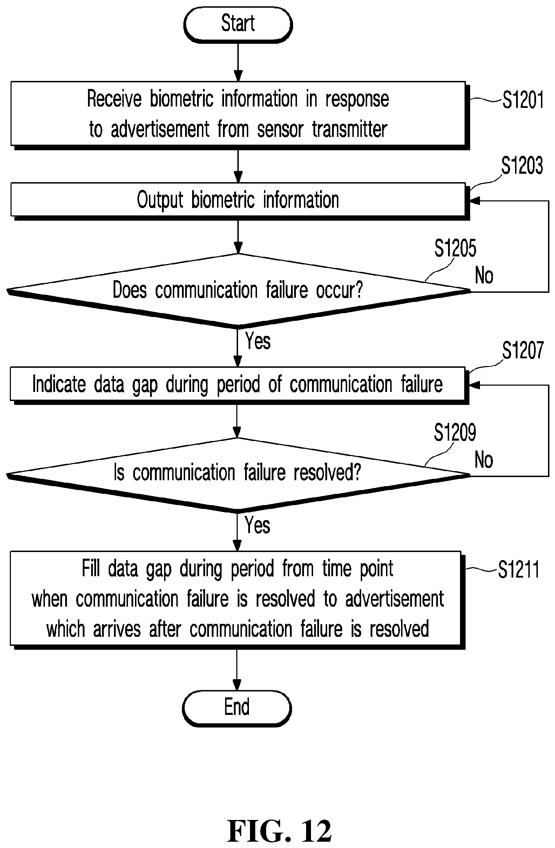

FIG. 12 is a flowchart illustrating an example of a method of receiving and outputting biometric information stored in a sensor transmitter by a terminal according to an embodiment.

Referring to FIG. 12, an example of a method of receiving and outputting biometric information stored in a sensor transmitter by a terminal according to an embodiment may be illustrated.

The controller of the terminal may receive biometric information from the sensor transmitter through the communicator in response to an advertisement (step S1201). The outputter of the terminal may visually output the biometric information to the user through a user interface (step S1203).

When a communication failure occurs, the controller of the terminal may detect and determine the communication failure through the communicator (step S1205). If it is determined that a communication failure has not occurred, the controller of the terminal may control the communicator and the outputter to continuously receive and output biometric information (NO of step S1205 and step S1203).

If it is determined that a communication failure has occurred, the controller of the terminal may control the outputter to output a data gap representing a blank of biometric information during a period in which the biometric information cannot be received (YES of step S 1205 and step S1207).

If the communication failure is resolved in the meantime, the controller of the terminal may detect and determine whether the communication failure is resolved through the communicator (step S1209). If it is determined that the communication failure is not resolved, the controller of the terminal may control the outputter to continuously output the data gap (NO of step S1209 and step S1207).

If it is determined that the communication failure is resolved, the terminal may receive and output biometric information stored in the sensor transmitter through the outputter to fill the data gap at a time point during the period from the time point when the communication failure is resolved to one advertisement timing which arrives after the communication failure is resolved (YES of step S1209 and step S1211).

Specifically, the terminal may go through the following process to receive biometric information stored in the sensor transmitter from the sensor transmitter. The sensor transmitter may basically generate 12 hours of biometric information and store it internally. The sensor transmitter may store biometric information without sending it to the terminal during the communication failure. Unreceived biometric information (unreceived data) may refer to biometric information that was not transmitted to the terminal during the communication failure.

If the communication failure is resolved, the sensor transmitter may send biometric information that was not transmitted during the communication failure to the terminal. The terminal may resume data transmission and reception with the sensor transmitter and request the sensor transmitter for biometric information that follows the biometric information that was last received before the communication failure. The terminal may send the identifier of the biometric information last received before the occurrence of the communication failure, for example, a serial number or sequence number assigned according to the order of generation, to the sensor transmitter. The sensor transmitter may transmit the biometric information assigned the next serial number or sequence number to the terminal. The terminal may receive the biometric information assigned the next serial number or sequence number. Here, even if the sensor transmitter cannot send the biometric information to the terminal due to a communication failure, the sensor transmitter may generate and store 12 hours worth of biometric information and send the stored biometric information to the terminal as unreceived data. If the communication failure exceeds 12 hours, the terminal may send the most recent biometric information excluding the biometric information of the earliest time. Therefore, the terminal cannot receive the excluded biometric information and may indicate a data gap.

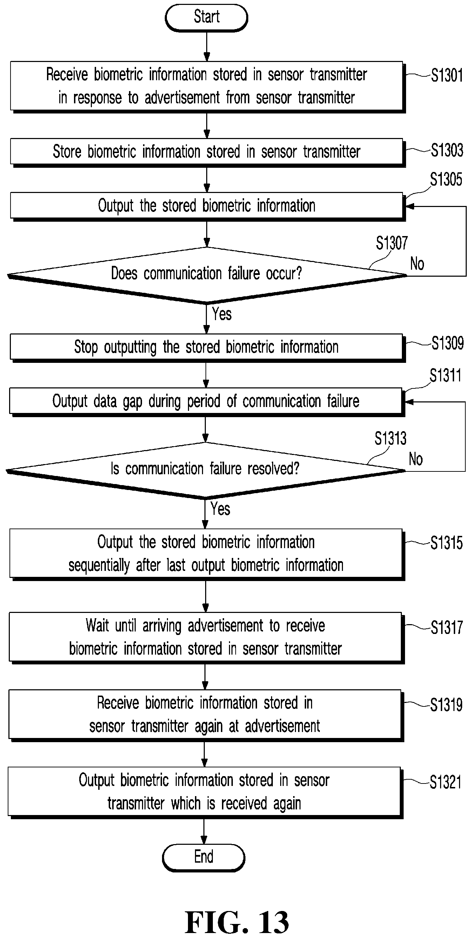

FIG. 13 is a flowchart illustrating another example of a method of receiving and outputting biometric information stored in a sensor transmitter by a terminal according to an embodiment.

Referring to FIG. 13, another example of a method of receiving and outputting biometric information stored in a sensor transmitter by a terminal according to an embodiment may be illustrated. Unlike the example described above, in this example, when the terminal receives biometric information stored in the sensor transmitter from the sensor transmitter, it does not output it immediately, but may first store the biometric information and then sequentially output the stored biometric information. In this case, while a communication failure occurs and is resolved, the terminal may output a data gap and again receive and output biometric information stored in the sensor transmitter.

Specifically, the controller of the terminal may receive biometric information from the sensor transmitter through the communicator in response to an advertisement (step S1301). The controller of the terminal may store the biometric information in an internal storage (step S1303). The controller of the terminal may sequentially read the stored biometric information and cause the outputter to visually output the stored biometric information to the user through a user interface (step S1305). Here, the stored biometric information may be sequentially output through the assigned identifier. The identifier may include a serial number or sequence number assigned in the order of generation in the sensor transmitter to indicate the temporal order.

When a communication failure occurs, the controller of the terminal may detect and determine the communication failure through the communicator (step S1307). If it is determined that the communication failure has not occurred, the controller of the terminal may control the storage to continuously receive and store biometric information (NO of step S1307 and step S1305).

If it is determined that a communication failure has occurred, the terminal cannot receive data, and the controller of the terminal may stop outputting the biometric information stored in the terminal during the period in which the biometric information is not received (YES of step S1307 and step S1309). In addition, the controller of the terminal may control the outputter to output a data gap during the period of the communication failure (step S1311).

If the communication failure is resolved, the controller of the terminal may detect and determine whether the communication failure is resolved through the communicator (step S1313). If it is determined that the communication failure is not resolved, the controller of the terminal may control the outputter to continuously output the data gap (NO of step S1313 and step S1311).

If it is determined that the communication failure is resolved, the terminal starts receiving data again, and the controller of the terminal may sequentially read the stored biometric information after the last output stored biometric information and output them through the outputter (YES of step S1313 and step S1315). If the output is stopped immediately after the communication failure occurs, the stored biometric information that has not been output may remain. If the communication failure is resolved, the biometric information following the last output stored biometric information may be output continuously. This output order may be identified by the identifier.

Then, if all the stored biometric information is output at one time point during the first period (T1 of FIG. 10), the terminal may wait to receive the subsequent biometric information from the sensor transmitter (step S1317). Since the terminal transmits and receives data at each advertisement timing, the terminal may wait for data re-reception up to this time point.

The communicator of the terminal may receive biometric information again at each advertisement timing including the first advertisement timing which arrives after the communication failure is resolved (step S1319). The terminal may receive biometric information stored in the sensor transmitter again to fill the data gap through the outputter, and output the re-received biometric information, i.e., biometric information stored in the sensor transmitter (step S1321).

The aspects of the subject matter described herein may be described in the context of computer-executable instructions, such as program modules, that are executed on a computer. Generally, program modules include routines, programs, objects, components, data structures, etc., which perform specific tasks or specific abstract data types.

Alternatively or additionally, the functionality described herein may be performed, at least in part, by one or more hardware logic components. By way of example, and not limitation, exemplary types of hardware logic components that may be used include field-programmable gate array (FPGA), program-specific integrated circuit (ASIC), application-specific standard product (ASSP), system-on-a-chip system (SOC), complex programmable logic device (CPLD), etc.

In addition, the disclosed embodiments may be implemented in the form of a non-transitory recording medium that stores programs and/or instructions executable by a computer. Instructions may be stored in the form of program code, and when executed by a processor, may create program modules to perform operations of the disclosed embodiments. The non-transitory recording medium may be implemented as a non-transitory computer-readable recording medium.

Computer-readable recording media include all types of recording media storing instructions that may be decoded by a computer. For example, there may be read only memory (ROM), random access memory (RAM), magnetic tape, magnetic disk, flash memory, optical data storage, etc.

Although embodiments of the present disclosure have been described above, those skilled in the art will be able to make various modifications and changes to the present disclosure by adding, changing or deleting components, without departing from the present disclosure as set forth in the appended claims, which are included within the scope of rights of the present disclosure.

## Claims

1. A method of outputting blood glucose data, the method comprising:
obtaining (S1201) biometric information in response to an advertisement transmitted by a sensor transmitter;
generating (S1203) the biometric information as output;
in the case that a communication failure occurs, indicating (S1207) a data gap during a period of the communication failure; and
if the communication failure is resolved, filling (S1211) the data gap during a period from a time point when the communication failure is resolved to one advertisement which arrives after the communication failure is resolved.

2. The method according to claim 1, wherein the filling (S1211) of the data gap comprises outputting biometric information stored in the sensor transmitter during the communication failure to fill the data gap after the communication failure is resolved.

3. The method according to claim 1, wherein the filling (S1211) of the data gap comprises outputting biometric information stored in the sensor transmitter during the communication failure to fill the data gap at an advertisement which arrives first after the communication failure is resolved.

4. The method according to claim 1, further comprising:
storing (S1303) biometric information stored in the sensor transmitter in a terminal,
wherein the filling (S1211) of the data gap comprises sequentially outputting biometric information stored in the terminal after last output biometric information.

5. The method according to claim 4, wherein the indicating (S1207) of the data gap comprises, in the case that the communication failure occurs, outputting the data gap while the biometric information stored in the terminal exists.

6. The method according to claim 4, wherein the filling (S 1211) of the data gap comprises, if the communication failure is resolved, outputting the biometric information stored in the terminal again following the last output biometric information.

7. The method according to claim 5, wherein the filling (S1211) of the data gap comprises, after sequentially outputting the biometric information stored in the terminal after the last output biometric information, waiting until an advertisement.
